(19) Europäisches Patentamt
European Patent Office
Office européen des brevets

(11) Publication number: **0 048 051**
**B1**

(12) **EUROPEAN PATENT SPECIFICATION**

(45) Date of publication of patent specification: **12.12.84**

(51) Int. Cl.$^3$: **C 07 C 103/52**, A 61 K 37/02, A 23 L 1/236

(21) Application number: **81200967.8**

(22) Date of filing: **01.09.81**

(54) **Dipeptide sweetener.**

(30) Priority: **04.09.80 NL 8005006**

(43) Date of publication of application:
**24.03.82 Bulletin 82/12**

(45) Publication of the grant of the patent:
**12.12.84 Bulletin 84/50**

(84) Designated Contracting States:
**BE CH DE FR GB IT LI NL SE**

(56) References cited:
**US-A-4 173 562**

(73) Proprietor: **STAMICARBON B.V.**
**Postbus 10**
**NL-6160 MC Geleen (NL)**

(72) Inventor: **Boesten, Wilhelmus Hubertus Joseph**
**Brountslaan 9**
**NL-6132 BJ Sittard (NL)**
Inventor: **Schiepers, Lambertus Albertus Christiaan**
**Galopiahof 28**
**NL-6215 TK Maastricht (NL)**

(74) Representative: **Hatzmann, Marinus Jan et al**
**OCTROOIBUREAU DSM P.O.Box 9**
**NL-6160 MA Geleen (NL)**

**EP 0 048 051 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to new dipeptide sweeteners and sweetening preparations based on these compounds.

Dipeptide sweeteners known in the art include L-alpha-aspartyl-L-phenylalaninemethylester, L-alpha-aspartyl-L-phenyulglycinemethylester and L-alpha-aspartyl-L-beta-cyclohexylalaninemethylester. A disadvantage of these dipeptide sweeteners is that they can form diketopiperazine derivatives.

In literature it has been stated that only the unprotected dipeptides are sweet. (see: Journal of Medicinal Chemistry 1970, Vol. 13, No. 6, page 1217 and ditto 1980, Vol. 23, No. 4, page 420).

The purpose of the invention is to obtain dipeptide sweeteners which cannot form diketopiperazine derivatives.

Now, it has been found that compounds with the formula I are sweeteners.

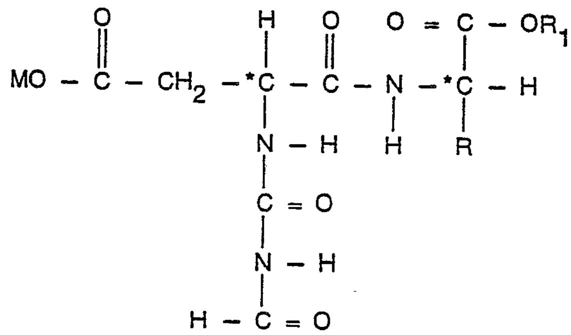

Formula I

where: M represents hydrogen, ammonium, alkali-, or alkaline earthmetalion,

R represents 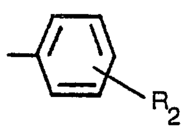 , 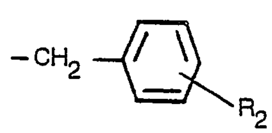 , 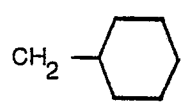 ,

$R_1$ represents methyl, ethyl, propyl,
$R_2$ represents H, —OH, —$OCH_3$,
* = L Configuration.

It has been found from organoleptic research that these compounds are about as sweet as the unprotected sweeteners similar to these compounds.

In Journal Med. Chemistry, Vol. 13, page 1217 (1970) it is stated that the unsubstituted amino group of the aspartic acid is necessary for the sweetness of L-alpha-aspartyl-L-phenylalaninemethylester. Journal Med. Chemistry, Vol. 23, page 420 (1980) describes the effects of hydroxy and methoxy substitution of the aromatic ring in L-alpha-aspartyl-L-phenylalaninemethylester. The importance of the unmodified peptide bond which connects the N-terminal zwitterionic part and the L-terminal hydrophobic portion is pointed out.

It is surprising that the N-(N′-formyl)carbamyl protected dipeptides found by the applicant are sweet.

The advantage of the N-protected dipeptide sweeteners and their salts is that these N-protected dipeptide sweeteners cannot form diketopiperazine derivatives. Hence the N-protected dipeptide sweeteners are more stable, and there is no loss of sweetening power by the formation of diketopiperazine derivatives which are not sweet.

The new compounds according to the invention can be obtained by reacting the unprotected dipeptides with an alkali cyanate and by treating the reaction product with the reaction product of acetic anhydride and formic acid. The N-(N-formyl)carbamyl derivatives can be prepared also by starting from N-(N′-formyl)carbamyl aspartic anhydride obtained by reaction of aspartic acid with alkali cyanate and by reacting the carbamyl compound obtained with the reacion product of formic acid and acetic anhydride. To react the product obtained with an alkyl ester of the amino acid, whether or not in the form of a salt, for instance as described in the Dutch application 7115944 laid open to public

inspection. Examples are: the N-(N'-formyl)carbamyl compounds of the esters of L-alpha-aspartyl-L-phenylalanine, L-alpha-aspartyl-L-phenyl-glycine, L-alpha-aspartyl-L-beta-cylcohexylalanine, L-alpha-aspartyl-L-tyrosine. Preference is given to N-(N'-formyl)carbamyl-L-alpha-aspartyl-L-phenylalanine-methylester on account of the good taste and the great sweetening power.

The invention also relates to a process for the sweetening of foods and allied-products, and pharmaceutical preparations comprising the addition of one or more of the above-mentioned N-(N'-formyl)carbamyl-protected dipeptides.

The N-(N'-formyl)carbamyl-protected dipeptides can be applied as such or be processed to sweetening preparations, with a pharmacologically acceptable carrier for instance in the form of tablets or solutions. These compounds can also be applied as sweetening product mixed with other sweeteners, such as saccharine and cyclamate, or with sugars, such as fructose.

The preparation of the new sweeteners is elucidated by means of the following examples.

Example I

In a flask (0.5 l) provided with a stirrer 8.5 g (0.029 Mol) L-alpha-aspartyl-L-phenylalanine-methylester was dissolved in a solution of 4.6 g (0.058 Mol) potassium cyanate in 100 ml water and subsequently stirred at room temperature for 24 hours. After that the turbidity obtained was filtered and the filtrate obtained was acidified with HCl to pH 2 and subsequently evaporated at 30°C and 12 mbar.

The deposit obtained was incorporated in 250 ml iso-propanol and evaporated again to dryness. This was repeated and the crystal mass obtained was incorporated in 500 ml isopropanol and subsequently stirred for 1 hour. The suspension was then filtered for the purpose of removing the KCl, and the filtrate obtained was evaporated and dried. 8.3 g (0.025 Mol) product was obtained, which, according to nuclear spin resonance (for spectra see figure 1) and infra-red analysis, consisted of N-carbamyl-L-alpha-aspartyl-L-phenyl-alaninemethylester. Yield 85%.

While being stirred at 25°C, 4 g (0.012 Mol) of this carbamyl compound was dissolved in a mixture of 20 ml acetic anhydride and 100 ml formic acid. At this temperature a reaction was effected for 18 hours, after which 3 ml water was added and subsequently evaporated to dryness.

The dry product was incorporated in 100 ml diethyl ether and subsequently filtered. The product obtained was washed on the filter with twice 50 ml diethyl ether and subsequently dried.

3.5 g (0.0096 Mol) product was obtained, consisting, according to nuclear spin resonance (for Spectra see figures 2, 3 and 4), mass spectrometry and infrared analysis, of N-(N'-formyl)carbamyl-L-alpha-aspartyl-L-phenylalaninemethylester. Yield 80%.

It was found from organoleptic research that this compound was 200 times sweeter than sucrose. No difference could be established with the sweetener L-alpha-aspartyl-L-phenylalanine-methylester.

Figure I is the 100 MHz $^1H$ Spectrum of N-carbamyl-L-alpha-aspartyl-L-phenylalaninemethylester dissolved in DMSO d6.

Figure 2 is the 100 MHz $^1H$ Spectrum of N-(N'-formyl)carbamyl-L-alpha-aspartyl-L-phenyl-alaninemethylester dissolved in DMSO d6.

Figure 3 shows the decoupling experiments at 60 MHz on N-(N'-formyl)carbamyl-L-alpha-aspartyl-L-phenylalaninemethylester in DMSO d6, the irradiation frequencies having been indicated with arrows.

Figure 4 is the 25.2 MHz $^{13}C$ spectrum of N-(N'-formyl)carbamyl-L-alpha-aspartyl-L-phenylalanine-methylester in DMSO d6, where a = $^1H$ decoupled and B = not decoupled.

Example II

In a flask (0.5 l) provided with a stirrer 4.3 g (0.015 Mol) L-alpha-aspartyl-L-phenylglycinemethylester was suspended, while being stirred, in a solution of 2.49 g (0.03 Mol) potassium cyanate in 125 ml water. After reacting for 18 hours at 20°C, filtration was effected.

The filtrate was brought to pH 2 by acidification with HCl, in which process crystallization commenced. After stirring for 15 minutes more filtration was carried out, and the crystal mass obtained was dried. 2.7 g solid product was obtained consisting, according to nuclear spin resonance and infra-red analysis, of N-carbamyl-L-alpha-aspartyl-L-phenylglycinemethylester. By extraction of the filtrate, 5 times with 100 ml ethyl acetate, and additional 0.9 g of carbamyl compound was obtained. Yield 74%.

1.3 g (0.004 Mol) of the N-carbamyl-L-alpha-aspartyl-L-phenylglycinemethylester was incorporated in a mixture of 10 ml acetic anhydride and 50 ml formic acid, upon which the whole was stirred for 20 minutes more at 25°C to dissolve everything. After reacting for 18 hours, 1.5 ml water was added, well stirred and subsequently evaporated to dryness at 30°C and 12 mbar. The product obtained was stirred with 25 ml diethyl ether and subsequently filtered. The crystal slurry was washed on the filter with 25 ml diethyl ether and subsequently dried.

1.2 g product was obtained consisting, according to nuclear spin resonance, mass spectrometry and infrared analysis, of N-(N'-formyl)carbamyl-L-alpha-aspartyl-L-phenylglycinemethylester. Yield 86%.

It was found from organoleptic research that this compound was 200 times sweeter than sucrose.

**Claims**

1. Compounds with the general formula

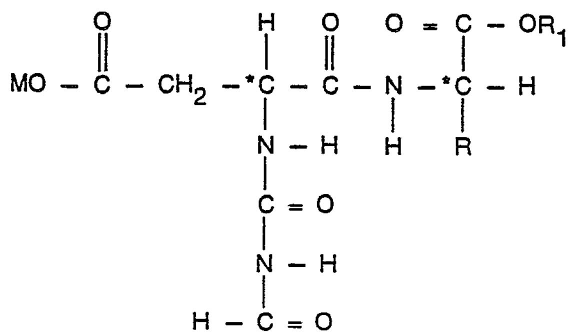

where M represents hydrogen, ammonium, alkali- or alkaline earth metal ion,

R represents 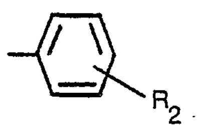 , 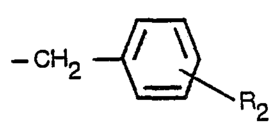 , 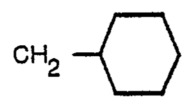

$R_1$ represents methyl, ethyl, propyl,
$R_2$ represents H—OH, —$OCH_3$,
* = L-configuration.

2. N-(N'-formyl)carbamyl L-alpha-aspartyl-L-phenylalaninemethylester.

3. N-(N'-formyl)carbamyl-L-alpha-aspartyl-L-phenylglycinemethylester.

4. Process for the sweetening of foods and allied-products, and pharmaceutical preparations characterized in that one or more compounds according to claims 1—3 are added.

5. Preparation with sweetening effect consisting of one or more compounds according to claims 1—3 and a pharmacologically acceptable carrier.

6. Preparation with sweetening effect consisting of one or more compounds according to claims 1—3 and one or more sweeteners from the group saccharine, cyclamate and fructose, possible with a pharmacologically acceptable carrier.

**Revendications**

1. Composés de formule générale:

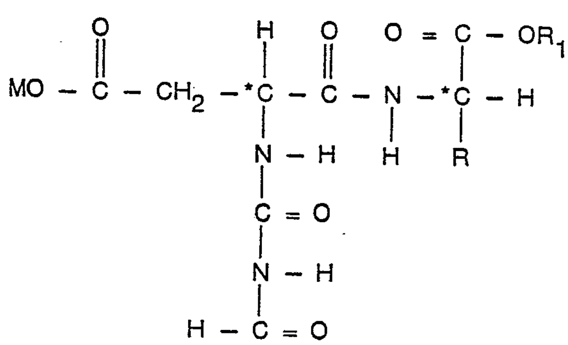

dans laquelle M représente un atome d'hydrogène, un ion ammonium, un ion de métal alcalin ou un ion de métal alcalino-terreux,

R représente 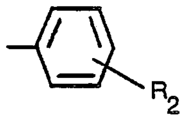 , 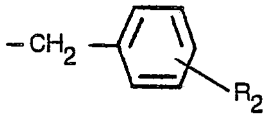 , 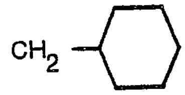

$R_1$ représente un groupe méthyle, éthyle, propyle,
$R_2$ représente H, —OH, —$OCH_3$,
* = configuration L.

2. N-(N'-formyl)-carbamyl-L-alpha-aspartyl-L-phénylalanineméthylester.

3. N-(N'-formyl)-carbamyl-L-alpha-aspartyl-L-phénylglycineméthylester.

4. Procédé pour édulcorer des aliments et des produits du même genre, et préparations pharmaceutiques, caractérisés en ce qu'un ou plusieurs composés selon les revendications 1—3 sont ajoutés.

5. Préparation avec effet édulcorant constituée d'un ou de plusieurs composés selon les revendications 1—3 et d'un support acceptable d'un point de vue pharmacologique.

6. Préparation avec effet édulcorant constituée d'un ou de plusieurs composés selon les revendications 1—3 et d'un ou de plusieurs édulcorants du groupe saccharine, cyclamate et fructose, éventuellement avec un support acceptable d'un point de vue pharmacologique.

**Patentansprüche**

1. Verbindungen mit der allgemeinen Formel

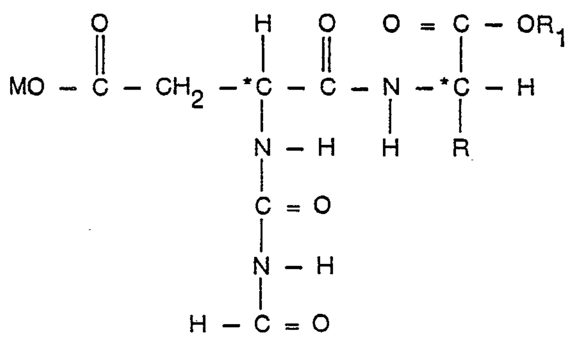

hierin steht M für Wasserstoff, Ammonium, Alkali oder Erdalkali Metallion,

R steht für 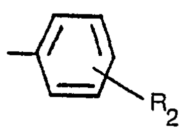 , 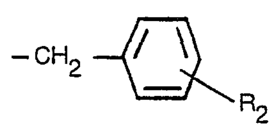 , 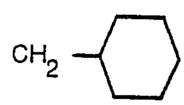

$R_1$ steht für Methyl, Ethyl, Propyl
$R_2$ steht für H, —OH, —$OCH_3$
* = L-Konfiguration.

2. N-(N'-Formyl)Carbamyl-L-alpha-Aspartyl-L-Phenylalaninemethylester.

3. N-(N'-Formyl)Carbamyl-L-alpha-Aspartyl-L-Phenylglyclinemethylester.

4. Verfahren zum Süssen von Lebensmitteln und verwandten Produkten sowie pharmazeutischen Präparaten, dadurch gekennzeichnet, dass eine oder mehrere Verbindungen nach den Ansprüchen 1—3 beigemischt werden.

5. Präparat mit süssender Wirkung, bestehend aus einer oder mehreren Verbindungen nach den Ansprüchen 1—3 und einem pharmakologisch akzeptablen Träger.

6. Präparat mit süssender Wirkung, bestehend aus einer oder mehreren Verbindungen nach den Ansprüchen 1—3 und einem oder mehreren süssenden Substanzen aus der Gruppe Saccharin, Cyclamat und Fruktosen, möglicherweise mit einem pharmakologisch akzeptablen Träger.

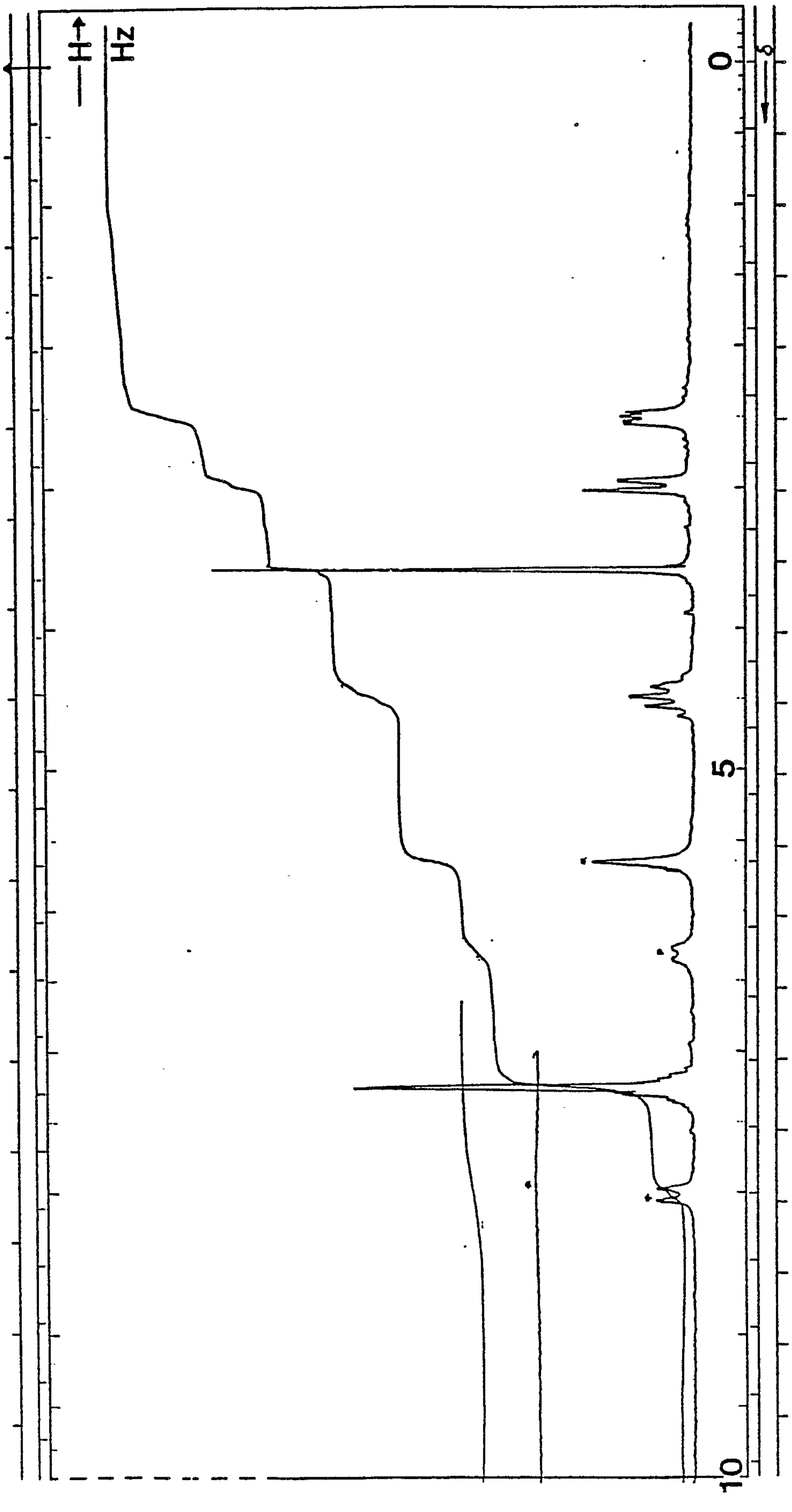
H
Hz
0
5
10
δ

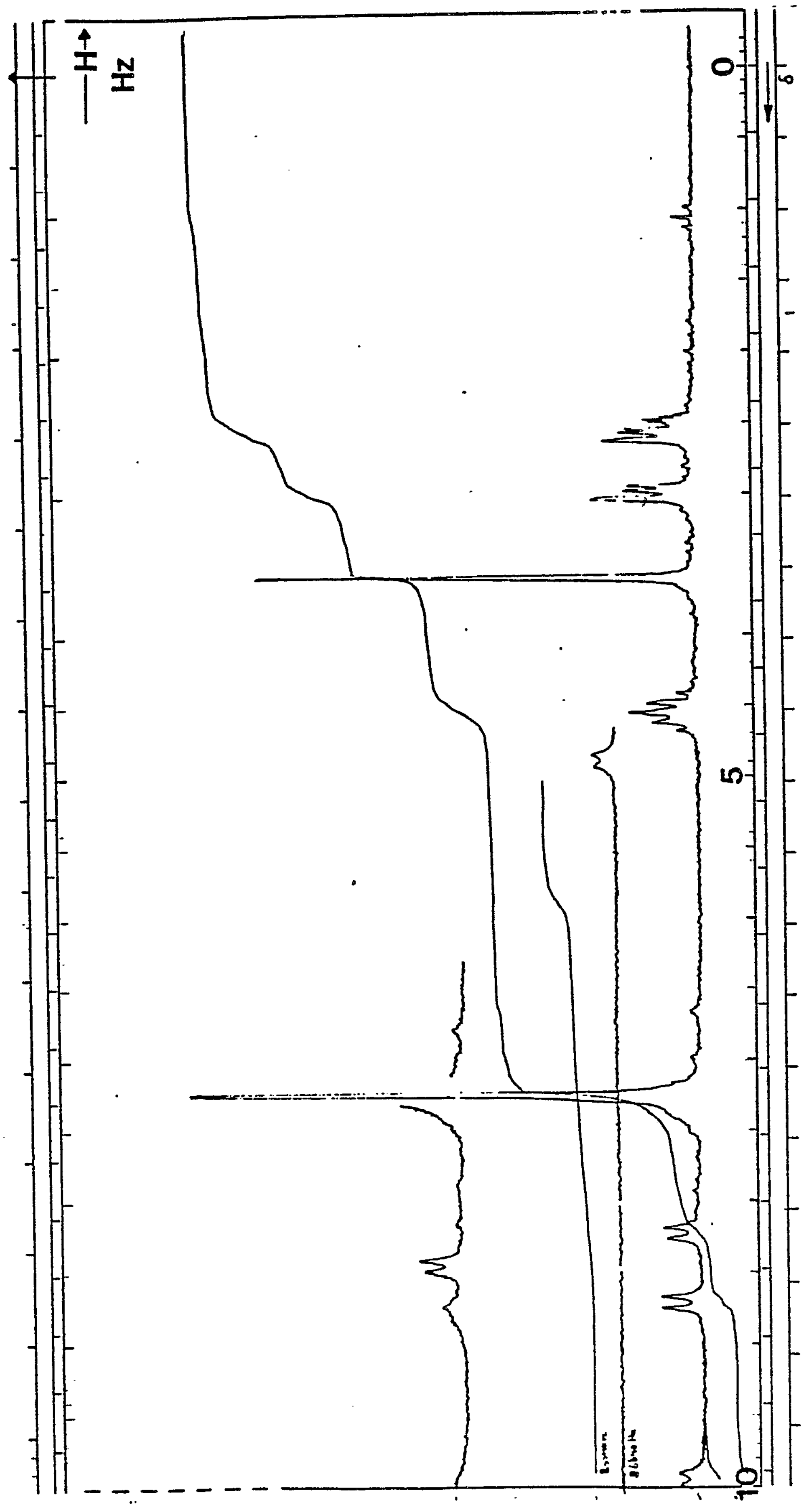
H
Hz
0
5
10
δ

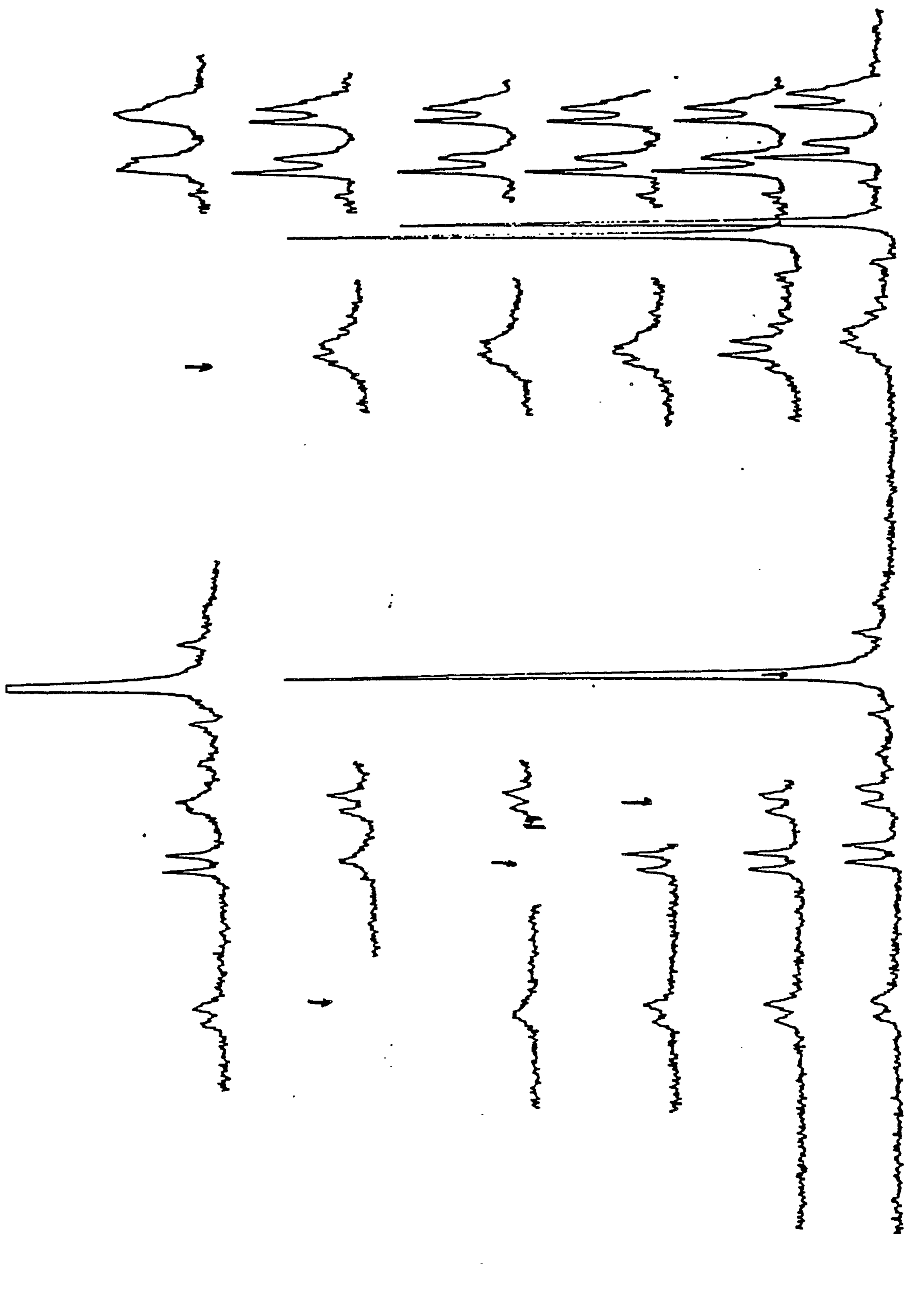

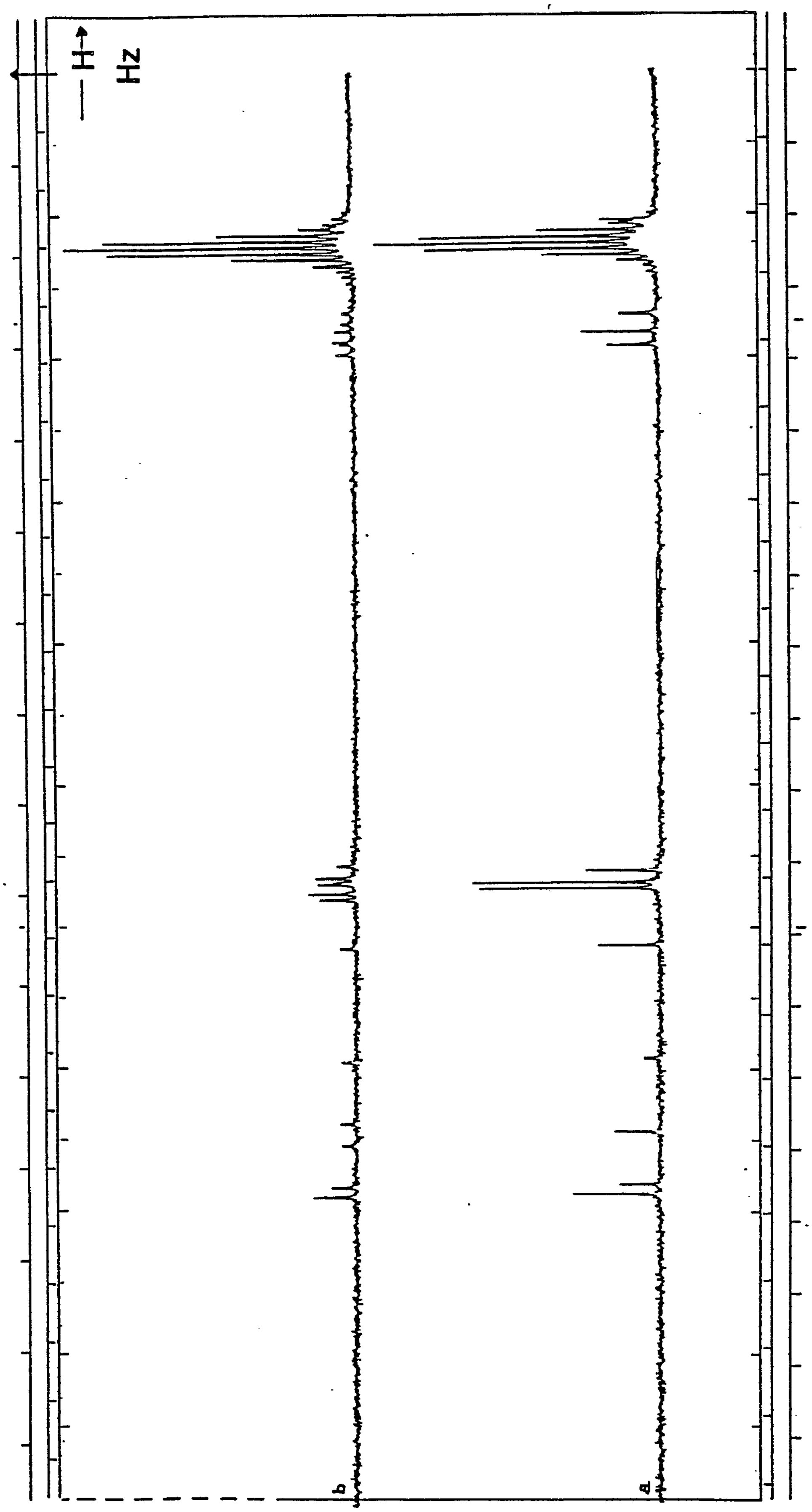
H
Hz
b
a